# EUROPEAN PATENT APPLICATION

(11) **EP 3 530 218 A1**
(43) Date of publication of application: **28.08.2019**
(21) Application number: 19159392.0
(22) Date of filing: 26.02.2019
(51) Int. Cl.: A61B 17/24, A61B 34/20, A61B 17/34

(54) **MEDICAL INSTRUMENT WITH SELF-COLLAPSING CHANNEL**

(30) Priority: 27.02.2018 US 201815906325
(71) Applicant: Acclarent, Inc., Irvine, CA 92618 (US)
(72) Inventor: PALUSHI, Jetmir, Irvine, CA 92618 (US); FANG, Itzhak, Irvine, CA 92618 (US); FARRINGTON, Ian, Irvine, CA 92618 (US); SHAMELI, Ehsan, Irvine, CA 92618 (US); SMITH, David A., Irvine, CA 92618 (US)
(74) Representative: Small, Gary James

(57) **Abstract**

An apparatus includes a cannula (562), a suction port (572), and a guidewire channel (580). The cannula includes a proximal end, a distal end, and a first lumen. The suction port is configured to communicate suction to the distal end of the cannula via the first lumen. The guidewire channel (580) includes an open proximal end, an open distal end, and a second lumen extending from the open proximal end to the open distal end. The guidewire channel is securely attached to the cannula such that the second lumen is laterally offset from the first lumen. The guidewire channel (580) is configured to slidably receive a guidewire (590) within the second lumen. The guidewire channel is formed of an elastic material such that the guidewire channel is configured to resiliently expand the second lumen outwardly to an expanded state in response to slidably receiving a guidewire therein.

## Description

### BACKGROUND

In some instances, it may be desirable to operate within or adjacent to an anatomical passageway of a patient, such as performing an incision of mucosa, removal of bone, or dilation of an anatomical passageway. Such operations may occur within anatomical passageways such as ostia of paranasal sinuses (e.g., to treat sinusitis), the larynx, the Eustachian tube, or other passageways within the ear, nose, or throat, etc. In addition to the above described operations, or similar operations, it may be desirable to apply suction and/or irrigation within or adjacent to an anatomical passageway before, during, or after the above described operations, or similar operations. One method of applying suction within or adjacent to an anatomical passageway of a patient involves obtaining a suction device having an elongate shaft defining a lumen terminating at an open distal end of the elongated shaft, where the lumen is in fluid communication with an external suction source. An operator may then insert the distal end of the elongate shaft within the nostril or mouth of a patient toward a desired location within the patient. With the distal end of the elongate shaft inserted within the patient, an operator may manipulate the suction device and/or suction source in order to remove extraneous and/or undesired matter near or within an anatomical passageway of a patient. Applying suction and/or irrigation during an operation may be beneficial for multiple purposes as will be apparent to those skilled in the art.

Image-guided surgery (IGS) is a technique where a computer is used to obtain a real-time correlation of the location of an instrument that has been inserted into a patient's body to a set of preoperatively obtained images (e.g., a CT or MRI scan, 3-D map, etc.), such that the computer system may superimpose the current location of the instrument on the preoperatively obtained images. In some IGS procedures, a digital tomographic scan (e.g., CT or MRI, 3-D map, etc.) of the operative field is obtained prior to surgery. A specially programmed computer is then used to convert the digital tomographic scan data into a digital map. During surgery, special instruments having sensors (e.g., electromagnetic coils that emit electromagnetic fields and/or are responsive to externally generated electromagnetic fields) mounted thereon are used to perform the procedure while the sensors send data to the computer indicating the current position of each surgical instrument. The computer correlates the data it receives from the instrument-mounted sensors with the digital map that was created from the preoperative tomographic scan. The tomographic scan images are displayed on a video monitor along with an indicator (e.g., crosshairs or an illuminated dot, etc.) showing the real-time position of each surgical instrument relative to the anatomical structures shown in the scan images. In this manner, the surgeon is able to know the precise position of each sensor-equipped instrument by viewing the video monitor even if the surgeon is unable to directly visualize the instrument itself at its current location within the body.

An example of an electromagnetic IGS systems that may be used in ENT and sinus surgery is the CARTO® 3 System by Biosense-Webster, Inc., of Irvine, California. When applied to functional endoscopic sinus surgery (FESS), balloon sinuplasty, and/or other ENT procedures, the use of IGS systems allows the surgeon to achieve more precise movement and positioning of the surgical instruments than can be achieved by viewing through an endoscope alone. As a result, IGS systems may be particularly useful during performance of FESS, balloon sinuplasty, and/or other ENT procedures where anatomical landmarks are not present or are difficult to visualize endoscopically.

While several systems and methods have been made and used in ENT procedures, it is believed that no one prior to the inventors has made or used the invention described in the appended claims.

### BRIEF DESCRIPTION OF THE DRAWINGS

While the specification concludes with claims which particularly point out and distinctly claim the invention, it is believed the present invention will be better understood from the following description of certain examples taken in conjunction with the accompanying drawings, in which like reference numerals identify the same elements and in which:
FIG. 1 depicts a schematic view of an exemplary sinus surgery navigation system being used on a patient seated in an exemplary medical procedure chair;
FIG. 2 depicts a perspective view of an exemplary suction instrument;
FIG. 3 depicts a cross-sectional side view of a proximal portion of the suction instrument of FIG. 2, the cross section taken along line 3-3 of FIG. 2;
FIG. 4 depicts a perspective view of an exemplary alternative suction instrument including an elastic guidewire channel, with the channel in a collapsed state;
FIG. 5 depicts a cross-sectional side view of an intermediate portion of the suction instrument of FIG. 4, the cross section taken along line 5-5 of FIG. 4;
FIG. 6 depicts a perspective view of the suction instrument of FIG. 4, with a guidewire received within the elastic guidewire channel;
FIG. 7 depicts a cross-sectional side view of an intermediate portion of the suction instrument of FIG 6 with a guidewire received within the elastic guidewire channel;
FIG. 8 depicts a side elevational view of an exemplary guidewire with a rigid distal portion;
FIG. 9 depicts a side elevational view of a portion of an exemplary alternative cannula with an elastic guidewire channel extending along a bent region of the cannula;
FIG. 10A depicts a side elevational view of the guidewire of FIG. 8 being inserted into the elastic guidewire channel of FIG. 9, with the rigid distal portion of the guidewire being positioned proximal to the bent region of the cannula of FIG. 9;
FIG. 10B depicts a side elevational view of the guidewire of FIG. 8 being further inserted into the elastic guidewire channel of FIG. 9, with the rigid distal portion of the guidewire being positioned at the bent region of the cannula of FIG. 9; and
FIG. 10C depicts a side elevational view of the guidewire of FIG. 8 being further inserted into the elastic guidewire channel of FIG. 9, with the rigid distal portion of the guidewire being positioned distal to the bent region of the cannula of FIG. 9.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the invention should not be used to limit the scope of the present invention. Other examples, features, aspects, embodiments, and advantages of the invention will become apparent to those skilled in the art from the following description, which is by way of illustration, one of the best modes contemplated for carrying out the invention. As will be realized, the invention is capable of other different and obvious aspects, all without departing from the invention. Accordingly, the drawings and descriptions should be regarded as illustrative in nature and not restrictive.

It will be appreciated that the terms "proximal" and "distal" are used herein with reference to a clinician gripping a handpiece assembly. Thus, an end effector is distal with respect to the more proximal handpiece assembly. It will be further appreciated that, for convenience and clarity, spatial terms such as "top" and "bottom" also are used herein with respect to the clinician gripping the handpiece assembly. However, surgical instruments are used in many orientations and positions, and these terms are not intended to be limiting and absolute.

It is further understood that any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

### I. Exemplary Image Guided Surgery Navigation System

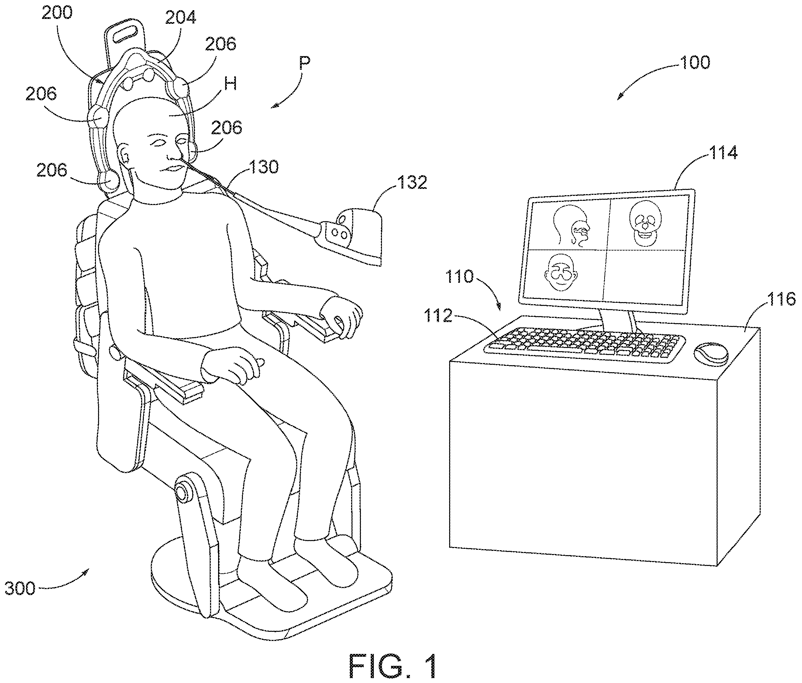

FIG. 1 shows an exemplary IGS navigation system (100) enabling an ENT procedure to be performed using image guidance. In addition to or in lieu of having the components and operability described herein IGS navigation system (100) may be constructed and operable in accordance with at least some of the teachings of U.S. Pat. No. 8,702,626, entitled "Guidewires for Performing Image Guided Procedures," issued April 22, 2014, the disclosure of which is incorporated by reference herein; U.S. Pat. No. 8,320,711, entitled "Anatomical Modeling from a 3-D Image and a Surface Mapping," issued November 27, 2012, the disclosure of which is incorporated by reference herein; U.S. Pat. No. 7,720,521, entitled "Methods and Devices for Performing Procedures within the Ear, Nose, Throat and Paranasal Sinuses," issued May 18, 2010, the disclosure of which is incorporated by reference herein; U.S. Pat. Pub. No. 2014/0364725, entitled "Systems and Methods for Performing Image Guided Procedures within the Ear, Nose, Throat and Paranasal Sinuses," published December 11, 2014, the disclosure of which is incorporated by reference herein; U.S. Pub. No. 2016/0310042, entitled "System and Method to Map Structures of Nasal Cavity," published October 27, 2016; and U.S. Pat. Pub. No. 2011/0060214, entitled "Systems and Methods for Performing Image Guided Procedures within the Ear, Nose, Throat and Paranasal Sinuses," published March 10, 2011, the disclosure of which is incorporated by reference herein.

IGS navigation system (100) of the present example comprises a field generator assembly (200), which comprises set of magnetic field generators (206) that are integrated into a horseshoe-shaped frame (204). Field generators (206) are operable to generate alternating magnetic fields of different frequencies around the head of the patient. Field generators (206) thereby enable tracking of the position of a navigation guidewire (130) that is inserted into the head of the patient. Various suitable components that may be used to form and drive field generators (206) will be apparent to those of ordinary skill in the art in view of the teachings herein.

In the present example, frame (204) is mounted to a chair (300), with the patient (P) being seated in the chair (300) such that frame (204) is located adjacent to the head (H) of the patient (P). By way of example only, chair (300) and/or field generator assembly (200) may be configured and operable in accordance with at least some of the teachings of U.S. Patent App. No. 62/555,824, entitled "Apparatus to Secure Field Generating Device to Chair," filed September 8, 2017, the disclosure of which is incorporated by reference herein.

IGS navigation system (100) of the present example further comprises a processor (110), which controls field generators (206) and other elements of IGS navigation system (100). For instance, processor (110) is operable to drive field generators (206) to generate electromagnetic fields; and process signals from navigation guidewire (130) to determine the location of a sensor in navigation guidewire (130) within the head (H) of the patient (P). Processor (110) comprises a processing unit communicating with one or more memories. Processor (110) of the present example is mounted in a console (116), which comprises operating controls (112) that include a keypad and/or a pointing device such as a mouse or trackball. A physician uses operating controls (112) to interact with processor (110) while performing the surgical procedure.

A coupling unit (132) is secured to the proximal end of a navigation guidewire (130). Coupling unit (132) of this example is configured to provide wireless communication of data and other signals between console (116) and navigation guidewire (130). While coupling unit (132) of the present example couples with console (116) wirelessly, some other versions may provide wired coupling between coupling unit (132) and console (116). Various other suitable features and functionality that may be incorporated into coupling unit (132) will be apparent to those of ordinary skill in the art in view of the teachings herein.

Navigation guidewire (130) includes a sensor (not shown) that is responsive to movement within the fields generated by field generators (206). In the present example, the sensor of navigation guidewire (130) comprises at least one coil at the distal end of navigation guidewire (130). When such a coil is positioned within an electromagnetic field generated by field generators (206), movement of the coil within that magnetic field may generate electrical current in the coil, and this electrical current may be communicated along the electrical conduit(s) in navigation guidewire (130) and further to processor (110) via coupling unit (132). This phenomenon may enable IGS navigation system (100) to determine the location of the distal end of navigation guidewire (130) within a three-dimensional space (i.e., within the head (H) of the patient (P)). To accomplish this, processor (110) executes an algorithm to calculate location coordinates of the distal end of navigation guidewire (130) from the position related signals of the coil(s) in navigation guidewire (130).

Processor (110) uses software stored in a memory of processor (110) to calibrate and operate system (100). Such operation includes driving field generators (206), processing data from navigation guidewire (130), processing data from operating controls (112), and driving display screen (114). Processor (110) is further operable to provide video in real time via display screen (114), showing the position of the distal end of navigation guidewire (130) in relation to a video camera image of the patient's head (H), a CT scan image of the patient's head (H), and/or a computer generated three-dimensional model of the anatomy within and adjacent to the patient's nasal cavity. Display screen (114) may display such images simultaneously and/or superimposed on each other during the surgical procedure. Such displayed images may also include graphical representations of instruments that are inserted in the patient's head (H), such as navigation guidewire (130), such that the operator may view the virtual rendering of the instrument at its actual location in real time. By way of example only, display screen (114) may provide images in accordance with at least some of the teachings of U.S. Pub. No. 2016/0008083, entitled "Guidewire Navigation for Sinuplasty," published January 14, 2016, the disclosure of which is incorporated by reference herein. In the event that the operator is also using an endoscope, the endoscopic image may also be provided on display screen (114).

The images provided through display screen (114) may help guide the operator in maneuvering and otherwise manipulating instruments within the patient's head. By way of example only, navigation guidewire (130) may facilitate navigation of instrumentation of dilation instrument assembly (10) within the patient during performance of a procedure to dilate the ostium of a paranasal sinus; to dilate some other anatomical passageway (e.g., within the ear, nose, or throat, etc.); and/or to perform one or more other procedures within the head (H) of the patient (P).

### II. Exemplary Suction Instrument Assembly

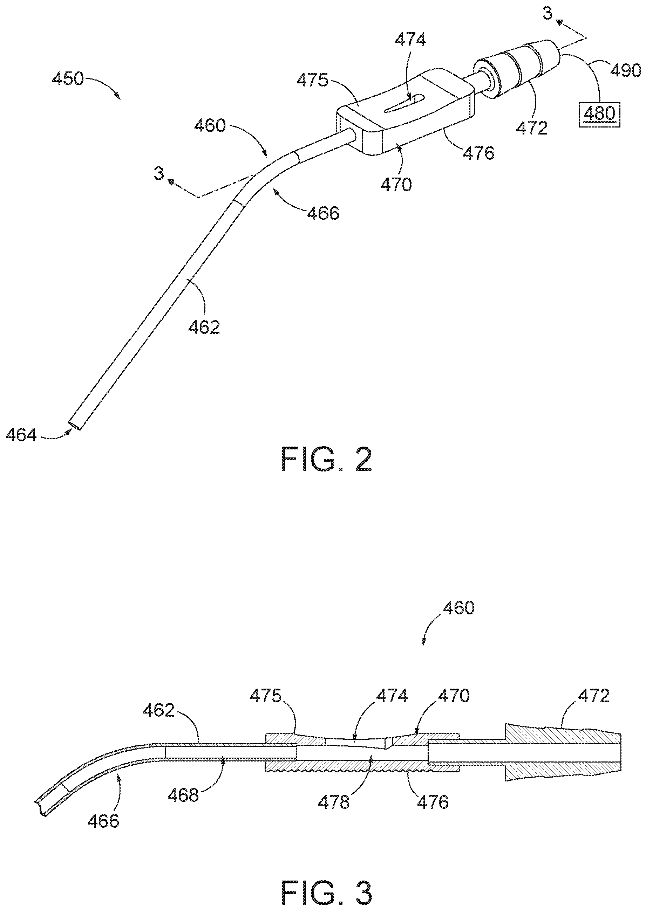

Various surgical procedures may warrant the use of a suction instrument to clear fluids and/or debris from the surgical field and/or from other sites within a patient. For instance, suction may be desirable in FESS procedures, sinuplasty procedures, and/or in various other ENT procedures. FIGS. 2-3 show an exemplary suction instrument assembly (450) that may be used to provide suction in such procedures. As shown, instrument assembly (450) includes a suction instrument (460) that is fluidly coupled with a suction source (480) via a conduit (490). Suction source (480) may comprise a vacuum pump and a fluid reservoir, among other components, as is known in the art. Suction source (480) is configured to provide enough suction to pull excess fluid and/or debris through suction instrument (460).

Suction instrument (460) of this example comprises an elongate cannula (462) extending distally from a grip portion (470). Cannula (462) has an open distal end (464) and a bent region (466) formed just distal to grip portion (470). Bent region (466) defines a bend angle that is selected to facilitate insertion of distal end (464) in a patient by an operator grasping grip portion (470). Various suitable bend angles that may be used will be apparent to those of ordinary skill in the art in view of the teachings herein.

In the present example, cannula (462) is rigid such that cannula (462) maintains the bend of bent region (466) and does not buckle during insertion into a patient's nasal cavity. By way of example only, cannula (462) may be formed of stainless steel (e.g., a stainless steel hypotube, etc.) and/or any other suitable rigid material. Also in the present example, cannula (462) defines a lumen (468) with a diameter of approximately 2.44 mm. Alternatively, any other suitable diameter may be used. It should also be understood that lumen (468) may have an elliptical cross-sectional profile or some other non-circular cross-sectional profile, if desired. A non-circular cross-sectional profile may provide additional clearance for other instruments to be positioned simultaneously in the same anatomical passageway (e.g., nasal cavity) with cannula (462). In some other variations, cannula (462) is malleable, such that the operator may bend cannula (462) to achieve a desired bend angle; with cannula (462) maintaining the desired bend angle during use in the nasal cavity (or elsewhere in the patient) to perform a suction procedure.

Grip portion (470) of the present example includes a proximal suction conduit port (472) that is configured to couple with conduit (490). In the present example, port (472) has a barbed configuration to promote a secure fit with an elastomeric conduit (490), though it should be understood that various other kinds of configurations may be used for port (472). Grip portion (470) of the present example further includes a transverse vent opening (474) formed through an upper surface (475); and a lower surface (476). As best seen in FIG. 3, vent opening (474) is in fluid communication with a lumen (478) formed through grip portion (470). Vent opening (474) has a teardrop shape in the present example, though it should be understood that vent opening (474) may have any other suitable shape. By way of example only, the teardrop shape (or some other elongate shape) may enable the operator to selectively vary the amount of suction based on the longitudinal position of the operator's thumb (or other finger) on vent opening (474). Lumen (478) is further in fluid communication with port (472) and a lumen (468) of cannula (462). It should be understood that lumens (468, 478) cooperate to provide an unobstructed fluid path from port (472) to open distal end (464) of cannula (462).

Surfaces (475, 476) are configured to promote gripping of grip portion (470) by an operator. In particular, upper surface (475) provides a concave contour while lower surface (476) provides a series of ridges. By way of example only, an operator may grasp grip portion (470) by placing a thumb on upper surface (475) and the side of the index finger of the same hand on lower surface (476). The rectangular shape of grip portion (470) may provide the operator with substantial purchase on grip portion (470), while the configurations of surfaces (475, 476) may further secure the operator's grip.

During use of suction instrument assembly (450), the operator may grasp grip portion (470) and position distal end (464) of cannula (462) at a target site in a patient. In some such instances, suction source (480) remains in a constantly activated state. In those instances, the operator may leave vent opening (474) uncovered as the operator positions instrument (460) relative to the patient. This may result in suction source (480) drawing suction through vent opening (474) without drawing suction through open distal end (464). When the operator wishes to apply the suction to the target site in the patient via open distal end (464), the operator may simply cover vent opening (474) with the operator's thumb (or otherwise cover vent opening (474)). The operator may thus selectively cover and uncover vent opening during a procedure in order to selectively apply suction.

While the above and below examples are provided in the context of suction instruments, it should be understood that the same instruments (and variations thereof) may be used to provide fluid irrigation at a target site in a patient; or to provide various other kinds of functionality. The teachings herein are thus not limited to suction instruments and operations per se. Other suitable instruments and procedures in which the teachings herein may be applied will be apparent to those of ordinary skill in the art. The various components and configurations of instrument (460) may also be modified in numerous ways as will be apparent to those of ordinary skill in the art in view of the teachings herein.

### III. Exemplary Alternative Suction Instrument with Elastic Guidewire channel

In some instances, it may be desirable to utilize a sensor-equipped navigation guidewire, like guidewire (130), at the target site in the patient while providing suction through a suction instrument, like suction instrument (460) described above. In conventional suction instruments that include a side channel, a sensor-equipped navigation guidewire like guidewire (130) might not fit in the side channel. In conventional suction instruments that include a larger sized lumen with a larger sized lumen with a substantial bend along its length (e.g. approximately 90 degrees), a rigid distal portion of a sensor-equipped navigation guidewire like guidewire (130) might not be able to traverse the substantial bend in the lumen. In conventional suction instruments that include a malleable cannula, the cannula may be susceptible to excessive bending once the navigation guidewire is received in the lumen of the cannula, thereby hindering the suction capabilities through the lumen of the cannula.

It may be beneficial to provide a surgical instrument, such as a modified version of suction instrument (460), that includes an elastic side channel defining a lumen that is capable of receiving a partially rigid navigation guidewire, like navigation guidewire (130), to preserve the functionality of the primary suction lumen (468) while allowing for navigational tracking of the distal end of the suction instrument within the patient. The elasticity of the side channel may accommodate the size of the outer diameter of the guidewire as the diameter of the lumen is selectively deformable to the size of the item being inserted therethrough. Furthermore, forming a channel from an elastic material may also provide as small of a cross sectional profile for the overall surgical instrument as possible since the side channel will conform to the size of the object received therein. Thus, in instances where the surgical instrument is intended to be used without a guidewire or other instrument received within the side channel, the side channel may collapse inwardly to reduce its respective profile and, as a result, the overall cross-sectional profile of the instrument cannula.

The following description provides various examples of a surgical instrument and corresponding elastic side channel that are cooperatively configured to assemble a guidewire onto surgical instrument. Ultimately, the capability of a surgical instrument to receive a sensor-equipped navigation guidewire at as minimal of a cross-sectional profile as possible may be beneficial to provide navigational date of the location of the surgical instrument during a surgical procedure while maintaining an atraumatic configuration of the instrument.

The elastic side channel described below may be readily incorporated into surgical instrument (460) described above, including any variations of surgical instrument (460) that will be apparent to those of ordinary skill in the art in view of the teachings herein. Moreover, the elastic side channel described below may be readily incorporated into various other kinds of medical instruments as will be apparent to those of ordinary skill in the art in view of the teachings herein. The following teachings are thus not limited to the context of suction instruments per se. Other suitable ways in which the below-described elastic side channels may be made and used will be apparent to those of ordinary skill in the art in view of the teachings herein.

### A. Exemplary Elastic Side Channel Extending Along Straight Portion of Cannula

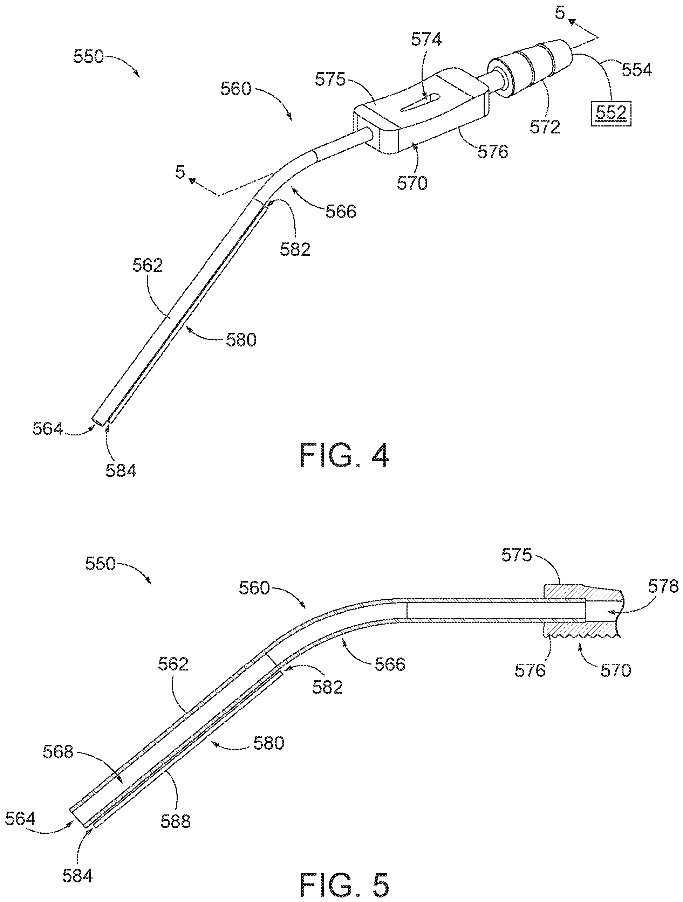

FIG. 4 shows an exemplary alternative suction instrument assembly (550) comprising a suction instrument (560) and a suction source (552). Except as otherwise described below, suction instrument assembly (550) and suction instrument (560) may be configured and operable just like suction instrument assembly (450) and suction instrument (460), respectively, described above. Suction instrument (560) is fluidly coupled with suction source (552) via a conduit (554), as similarly described above. Thus, suction source (552) is configured to provide enough suction to pull excess fluid and/or debris through suction instrument (560).

Similar to suction instrument (460) described above, suction instrument (560) of this example comprises an elongate cannula (562) extending distally from a grip portion (570). Cannula (562) has an open distal end (564) and a bent region (566) formed just distal to grip portion (570). Bent region (566) defines a bend angle that is selected to facilitate insertion of distal end (564) in a patient by an operator grasping grip portion (570). Various suitable bend angles that may be used will be apparent to those of ordinary skill in the art in view of the teachings herein.

Cannula (562) is rigid such that cannula (562) maintains the bend of bent region (566) and does not buckle during insertion into a patient's nasal cavity. By way of example only, cannula (562) may be formed of stainless steel (e.g., a stainless steel hypotube, etc.) and/or any other suitable rigid material. Also in the present example, cannula (562) defines a lumen (568) with a diameter of approximately 2.44 mm. Alternatively, any other suitable diameter may be used. It should also be understood that lumen (568) may have an elliptical cross-sectional profile or some other non-circular cross-sectional profile, if desired. A non-circular cross-sectional profile may provide additional clearance for other instruments to be positioned simultaneously in the same anatomical passageway (e.g., nasal cavity) with cannula (562). In some other variations, cannula (562) is malleable, such that the operator may bend cannula (562) to achieve a desired bend angle; with cannula (562) maintaining the desired bend angle during use in the nasal cavity (or elsewhere in the patient) to perform a suction procedure.

Grip portion (570) of the present example includes a proximal suction conduit port (572) that is configured to couple with conduit (554). In the present example, port (572) has a barbed configuration to promote a secure fit with an elastomeric conduit (554), though it should be understood that various other kinds of configurations may be used for port (572). Grip portion (570) of the present example further includes a transverse vent opening (574) formed through an upper surface (575); and a lower surface (576). As best seen in FIG. 5, vent opening (574) is in fluid communication with a lumen (578) formed through grip portion (570). Vent opening (574) has a teardrop shape in the present example, though it should be understood that vent opening (574) may have any other suitable shape. By way of example only, the teardrop shape (or some other elongate shape) may enable the operator to selectively vary the amount of suction based on the longitudinal position of the operator's thumb (or other finger) on vent opening (574). Lumen (578) is further in fluid communication with port (572) and a lumen (568) of cannula (562). It should be understood that lumens (568, 578) cooperate to provide an unobstructed fluid path from port (572) to open distal end (564) of cannula (562).

Surfaces (575, 576) are configured to promote gripping of grip portion (570) by an operator. In particular, upper surface (575) provides a concave contour while lower surface (576) provides a series of ridges. By way of example only, an operator may grasp grip portion (570) by placing a thumb on upper surface (575) and the side of the index finger of the same hand on lower surface (576). The rectangular shape of grip portion (570) may provide the operator with substantial purchase on grip portion (570), while the configurations of surfaces (575, 576) may further secure the operator's grip.

Suction instrument (560) further comprises a guidewire channel (580) securely attached to the side of cannula (562) such that guidewire channel (580) extends parallel to and external of the longitudinal length of cannula (562). In other words, guidewire channel (580) extends parallel to a longitudinal axis (not shown) defined by the longitudinal length of cannula (562) that is distal to bent region (566). Accordingly, an axis of guidewire channel (580) is coaxially offset from the longitudinal axis of cannula (562). Guidewire channel (580) is securely attached to cannula (562) by an engagement mechanism (not shown) to fixedly secure guidewire channel (580) along the longitudinal length of cannula (562). By way of example only, guidewire channel (580) may be secured to cannula (562) by means of an adhesive (e.g. glue, etc.), fastener, clips, and/or other suitable engagement features as will be apparent to those of ordinary skill in the art. In some versions, guidewire channel (580) may be releasably secured to cannula (562) such that guidewire channel (580) may be selectively disengaged from attachment with cannula (562).

Guidewire channel (580) has an open distal end (584) that is positioned adjacent to open distal end (564) of cannula (562). Guidewire channel (580) further includes an open proximal end (582) that is positioned opposite of open distal end (584) and proximate to (yet distal to) bent region (566) of cannula (562). In some other versions, open distal end (584) is positioned proximal to bent region (566) of cannula (562). In some such versions, guidewire channel (580) follows the same bend as bent region (566) of cannula (562).

Guidewire channel (580) defines a lumen (588) extending between open ends (582, 584). As will be described in greater detail below, although guidewire channel (580) is shown as having a substantially straight configuration, guidewire channel (580) may include one or more bends between open proximal end (582) and open distal end (584). As will also be described in greater detail below, although lumen (588) of guidewire channel (580) has a variable size and profile, lumen (588) is generally sized to removably receive a guidewire therein.

Guidewire channel (580) is resiliently biased inwardly into lumen (588) such that guidewire channel (580) is configured to elastically contract radially inwardly when a radially outward force is not exerted from within lumen (588). In other words, guidewire channel (580) is operable to resiliently collapse inwardly to minimize the effective outer diameter of cannula (562) and guidewire channel (580) when guidewire channel (580) is not in receipt of an object (e.g., navigation guidewire (130), etc.) within lumen (588). Thus, guidewire channel (580) has an effectively negligible diameter when in a default contracted or collapsed state, as shown in FIGS. 4-5. In this state, the diameter of lumen (588) is less than the outer diameter or cross-sectional profile of a guidewire such as navigation guidewire (130).

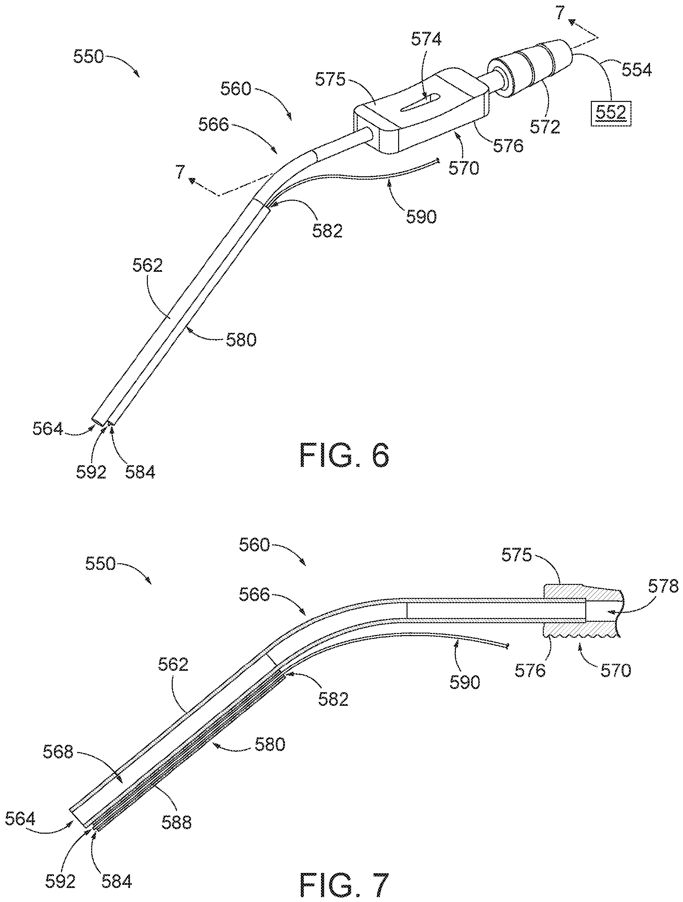

Guidewire channel (580) is also deformable such that guidewire channel (580) is configured to elastically deform outwardly when a radially outward force is applied from within lumen (588). In this instance, lumen (588) of guidewire channel (580) is operable to increase in diameter in response to a radially outward force being exerted within lumen (588). For instance, as seen in FIG. 6, a radially outward force may be introduced within lumen (588) by inserting a guidewire (590) into guidewire channel (580), thereby expanding guidewire channel (580) outwardly to an expanded state. Guidewire channel (580) is configured to slidably receive guidewire (590) through open proximal end (582) and into lumen (588). Guidewire (590) of this example may be configured and operable just like guidewire (130) described above, such that guidewire (590) includes one or more sensors that interact with electromagnetic fields generated by field generators (206), to thereby provide IGS navigation capabilities via IGS system (100).

As best seen in FIG. 7, the elasticity of guidewire channel (580) provides for the expansion of lumen (588) with guidewire (590) positioned therein. Open distal end (584) is configured to permit a distal end (592) of guidewire (590) to extend distally outwardly from lumen (588) such that distal end (592), where a navigational sensor (not shown) of guidewire (590) is located, is positioned adjacent to open distal end (564) of cannula (562).

While the above examples of guidewire channel (580) is provided in the context of suction instrument (560), the same channel (and variations thereof) may be used to provide other surgical instruments with an elastic guidewire channel coupled to a cannula of the instrument to provide the IGS navigational functionality described herein. The teachings herein are thus not limited to suction instruments and suction procedures. Other suitable instruments and procedures in which the teachings herein may be applied will be apparent to those of ordinary skill in the art in view. By way of example only, guidewire channel (580) may be secured onto the shafts of instruments such as curettes, seekers, shavers, and/or other various suitable surgical instruments.

During use of suction instrument assembly (550), an operator may insert guidewire (590) into guidewire channel (580) prior to inserting distal end (560) of cannula (562) into a target site in a patient. Alternatively, guidewire (590) may be inserted into guidewire channel (580) at any point during the procedure, including after distal end (564) of cannula (562) is located at the target site. Distal end (592) of guidewire (590) is advanced toward open proximal end (582) of guidewire channel (580) to thereby advance distal end (592) through proximal end (582) and into lumen (588). The elasticity of proximal end (582) accommodates the relatively rigid configuration of distal end (592) such that an effective diameter of proximal end (582) increases to thereby enable guidewire (590) to enter lumen (588). The operator advances guidewire (590) through lumen (588) until distal end (592) reaches open distal end (584) of guidewire channel (580).

As seen in FIGS. 5 and 7, lumen (588) is effectively expanded due to the radially outward force applied to guidewire channel (580) by the presence of guidewire (590) in lumen (588). Due to the deformability of guidewire channel (580), guidewire (590) is capable of being advanced through lumen (588) despite lumen (588) initially defining a smaller diameter than the outer diameter of guidewire (590). Distal end (592) of guidewire (590) extends through open distal end (584) of guidewire channel (580) to thereby position the sensor (not shown) of guidewire (590) adjacent to open distal end (564) of cannula (562).

With guidewire (590) effectively assembled to suction instrument (560), the operator may then grasp grip portion (570) and position distal end (564) of cannula (562) at a target site in a patient. The operator may observe display screen (114) of IGS navigation system (100) as the operator positions cannula (562) in the patient. In this instance, with distal end (592) of guidewire (590) being securely positioned adjacent to distal end (564) of cannula (562), the sensor of guidewire (590) is thereby simultaneously positioned at the target site in the patient. Display screen (114) may show the real-time position of distal end (564) of cannula (562) (e.g., superimposed on one or more CT scan images of the head (H) of the patient (P) as the operator positions cannula (562) in the patient, with the real-time position being established by processor (110) based on data from the sensor in navigation guidewire (590).

In some instances, suction source (552) remains in a constantly activated state as the operator positions cannula (562) in the patient. In those instances, the operator may leave vent opening (574) uncovered as the operator positions instrument (560) relative to the patient. This may result in suction source (552) drawing suction through vent opening (574) without drawing suction through open distal end (564). When the operator wishes to apply the suction to the target site in the patient via open distal end (564), the operator may simply cover vent opening (574) with the operator's thumb (or otherwise cover vent opening (574)). The operator may thus selectively cover and uncover vent opening during a procedure in order to selectively apply suction.

After the suction procedure is complete, the operator may pull guidewire (590) from channel (580). The elasticity of channel (580) may cause channel (580) to return to the contracted state after guidewire (590) is pulled from channel (580).

In some settings, an operator may wish to use suction instrument (560) without guidewire (590). In such scenarios, the operator may simply use suction instrument (560) in accordance with the teachings herein, with suction instrument (560) being in the state shown in FIGS. 6-7. In other words, suction instrument (560) may be readily used with channel (580) in the collapsed state, without guidewire (590) being disposed in channel (580).

In the example shown in FIGS. 4-7, channel (580) is shown as having a straight configuration, running parallel to the straight portion of cannula (562). In some such versions, the entire length of channel (580) is secured to cannula (562).

In some other versions, channel (580) has a bent configuration. For instance, proximal end (582) of channel (580) may be proximal to bent region (566) of cannula (562). In some such versions, channel (580) may follow the same bend contour of bent region (566); and the entire length of channel (580) may be secured to cannula (562). In some other versions, a longitudinally intermediate portion of channel (580) may be laterally separated from cannula (562), such that channel (580) need not necessarily follow the same bend contour of bent region (566). For instance, proximal end (582) of channel (580) may be secured to cannula (562) at a location proximal to bent region (566), and a distal portion of channel (580) (including distal end (584)) may be secured to cannula (562) at a location distal to bent region (566), with a longitudinally intermediate portion of channel (580) being laterally separated from cannula (562) at bent region (566). Such an arrangement may allow channel (580) to provide a less tortuous path for guidewire (560), such that channel (580) may more readily accommodate insertion of guidewire (560) than channel (580) might otherwise be in the event that channel (580) were to follow the same bend contour as cannula (562).

As yet another merely illustrative variation, the distal portion of cannula (562) may include one or more bends, rather than having the straight configuration shown in FIGS. 4-7. In such variations, the distal portion of channel (580) may be fully secured to cannula (562) along such bends, such that the distal portion of channel (580) follows the same bend contours as the distal portion of cannula (562). Alternatively, one or more distal portions of channel (580) may be laterally separated from the bend distal portion(s) of cannula (562), such that the separated distal portion(s) of channel (580) need not follow the same bend contours as the distal portion of cannula (562). As noted above, this separation may make channel (580) more accommodating to guidewire (560) than channel (580) might otherwise be in the event that distal portions of channel (580) were to follow the same bend contours as distal portions of cannula (562). Even in scenarios where one or more distal portions of channel (580) are laterally separated from cannula (562), distal end (584) of channel (580) may still be secured to distal end (564) of cannula (562). This may ensure proximity between distal end (592) of guidewire (590) and distal end (564) of cannula (562), thereby ensuring that the position data associated with distal end (592) of guidewire (590) is reliably representative of the position of distal end (564) of cannula (562) in three-dimensional space.

### B. Exemplary Elastic Side Channel Secured Along Bent Portion of Cannula

As noted above, there may be versions where channel (580) is secured along a bent region (566) of cannula (562). This may be particularly beneficial where a portion of guidewire (590) that needs to pass through channel (580) along bent region (566) is rigid. In the absence of an elastic channel (580), a rigid portion of a guidewire may be unable to traverse a bent region of cannula (562). In the example described below, an elastic channel yields along a bent region of a cannula to thereby enable a rigid portion of a guidewire to traverse along the bent region of the cannula.

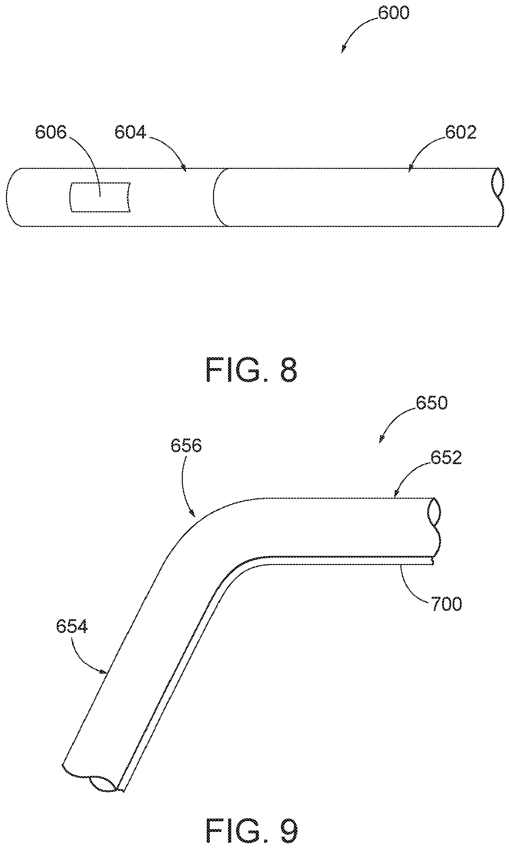

FIG. 8 shows an example of a guidewire (600) that includes a flexible portion (602) and a rigid distal end portion (604). In this example, a sensor (606) is located in rigid distal end portion (604). Guidewire (600) is configured and operable like guidewires (130, 190) described above, such that sensor (606) is responsive to movement within the electromagnetic fields generated by field generators (206) to thereby generate signals indicative of the position of distal end portion (604) within the head (H) of the patient (P).

FIG. 9 shows an exemplary cannula (650) having a first straight portion (652), a second straight portion (654), and a bent portion (656) longitudinally interposed between straight portions (652, 654). Cannula (650) may be incorporated into suction instrument (560) in place of cannula (562). Alternatively, cannula (650) may be part of any other kind of instrument where an operator may wish to effectively couple guidewire (600) with the instrument. While FIG. 9 shows bent portion (656) as defining a particular bend angle, bent portion (656) may instead have any other suitable bend angle (e.g., approximately 90°, greater than 90°, less than 90°). An elastic guidewire channel (700) is secured along the length of bent portion (656) and along at least part of each straight portion (652, 654). While the proximal and distal ends of cannula (650) are not shown in FIG. 9, some versions of guidewire channel (700) may extend along the entire length of cannula (650), from the proximal end of cannula (650) to the distal end of cannula (650). Alternatively, guidewire channel (700) may extend along any other portion of the length of cannula (650), provided that guidewire channel (700) extends at least along bent portion (656) in the present example.

In use, an operator may wish to insert guidewire (600) into guidewire channel (700) and traverse the entire length of guidewire channel (700) to position distal portion (604) of guidewire (600) at the distal end of cannula (650). In the present example, this requires rigid distal portion (604) to traverse along bent portion (656) of cannula (650). The elasticity of guidewire channel (700) allows guidewire channel (700) to yield along bent portion (656) to thereby accommodate rigid distal portion (604) traversing along bent portion (656) of cannula (650).

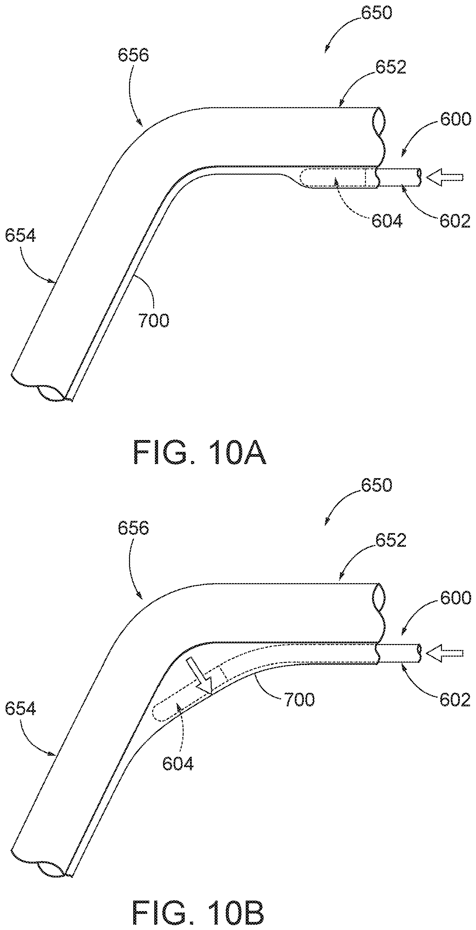

FIG. 10A shows guidewire (600) in a first longitudinal position where rigid distal portion (604) is located in a region of guidewire channel (700) along first straight portion (652) of cannula (650). As shown, guidewire channel (700) expands radially outwardly to accommodate the outer diameter of guidewire (600). The portion of guidewire channel (700) that is distal to guidewire (600) remains in the elastically contracted state, thereby presenting a much smaller cross-sectional profile.

FIG. 10B shows guidewire (600) advanced distally through channel (700) to a point where rigid distal portion (604) is adjacent to bent portion (656) of cannula (650). In this example, due to the length of rigid distal portion (604), the bend angle of bent portion (656), and the rigidity of cannula (650), channel (700) must deform in order to enable rigid distal portion (604) to traverse along bent portion (656). Channel (700) deforms accordingly, expanding away from bent portion (656) to accommodate rigid distal portion (604) as rigid distal portion (604) travels from alongside first straight portion (652) of cannula (650) to second straight portion (654) of cannula (650).

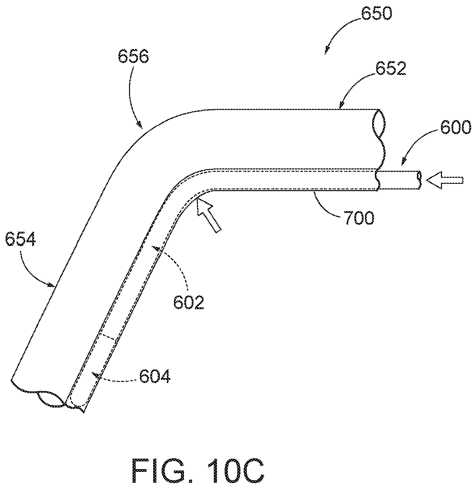

After rigid distal portion (604) clears bent portion (656) of cannula (650), rigid distal portion (604) is positioned only alongside second straight portion (654) of cannula (650); and flexible portion (602) of guidewire (600) is positioned alongside bent portion (656) of cannula (650) as shown in FIG. 10C. In this example, the elastic bias of channel (700) causes channel (700) contract back toward bent portion (656), such that channel (700) and flexible portion (602) both substantially confirm to the bend of bent portion (656). This conformance is merely optional and need not necessarily occur in every scenario. In either case, those of ordinary skill in the art will recognize that the presence of channel (700), and the elasticity of channel (700) have accommodated rigid distal portion (604) of guidewire (600) the bent portion (656) of cannula (650) while channel (700) substantially secures guidewire (600) alongside cannula (650).

In the foregoing examples, cannulas (562, 650) and channels (580, 700) may be disposed of after a single use. Alternatively, cannulas (562, 650) and/or channels (580, 700) may be cleaned and otherwise reprocessed for subsequent uses.

### IV. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus comprising: (a) a cannula including: (i) a proximal end, (ii) a distal end, and (iii) a first lumen extending from the proximal end to the distal end; (b) a suction port that is configured to communicate suction to the distal end of the cannula via the first lumen; and (c) a guidewire channel including: (i) an open proximal end, (ii) an open distal end, and (iii) a second lumen extending from the open proximal end to the open distal end, wherein the guidewire channel is securely attached to the cannula such that the second lumen is laterally offset from the first lumen, wherein the guidewire channel is configured to slidably receive a guidewire within the second lumen, wherein the guidewire channel is formed of an elastic material such that the guidewire channel is configured to resiliently expand the second lumen outwardly to an expanded state in response to slidably receiving a guidewire therein.

### Example 2

The apparatus of Example 1, wherein the guidewire channel is resiliently biased toward an inwardly contracted state.

### Example 3

The apparatus of Example 2, wherein the second lumen is configured to have a smaller cross-sectional profile than a guidewire when the guidewire channel is in the inwardly contracted state.

### Example 4

The apparatus of any one or more of Examples 1 through 3, wherein the guidewire channel is configured to elastically conform to a shape of a guidewire that is slidably received within the second lumen.

### Example 5

The apparatus of any one or more of Examples 1 through 4, wherein the open distal end of the guidewire channel is positioned adjacent to the distal end of the cannula.

### Example 6

The apparatus of any one or more of Examples 1 through 5, wherein the cannula further includes a preformed bend formed between the proximal end and the distal end.

### Example 7

The apparatus of Example 6, wherein the open proximal end of the guidewire channel is positioned adjacent to the preformed bend.

### Example 8

The apparatus of Example 6, wherein the guidewire channel further includes a bend formed between the open proximal end and the open distal end, wherein the bend is positioned adjacent to the preformed bend of the cannula.

### Example 9

The apparatus of any one or more of Examples 6 through 7, wherein the wherein the open proximal end of the guidewire channel is positioned distal to the preformed bend.

### Example 10

The apparatus of any one or more of Examples 1 through 9, wherein the first lumen defines a longitudinal axis, wherein the second lumen is oriented parallel relative to the longitudinal axis.

### Example 11

The apparatus of any one or more of Examples 1 through 10, wherein the cannula is rigid.

### Example 12

The apparatus of any one or more of Examples 1 through 11, further comprising a grip secured to the proximal end of the cannula, wherein the grip defines a third lumen providing a path for communication of suction from the suction port to the first lumen.

### Example 13

The apparatus of Example 12, wherein the guidewire channel is oriented obliquely relative to the third lumen.

### Example 14

The apparatus of any one or more of Examples 1 through 13, further comprising a guidewire configured to fit in the second lumen.

### Example 15

The apparatus of Example 14, wherein the guidewire includes a navigation sensor configured to communicate with an image guided surgery system.

### Example 16

An apparatus comprising: (a) a cannula including: (i) a proximal end, (ii) a distal end, and (iii) a first lumen extending from the proximal end to the distal end; (b) a guidewire channel securely attached to the cannula, wherein the guidewire channel defines a second lumen laterally offset from the first lumen, wherein the guidewire channel is configured to resiliently bias the second lumen inwardly to a contracted state; and (c) a navigation guidewire configured to fit in the second lumen such that the second lumen is operable to expand outwardly to an expanded state, wherein the navigation guidewire includes a sensor configured to generate data based on a location of the sensor within a patient; wherein the first lumen is configured to provide suction through the distal end while the navigation guidewire is disposed in the second lumen.

### Example 17

The apparatus of Example 16, wherein the guidewire channel is formed of an elastic material to provide the resilient bias.

### Example 18

The apparatus of any one or more of Examples 16 through 17, further comprising an image guided surgery system in communication with the sensor of the guidewire, wherein the image guided surgery system is configured to process the data from the sensor to determine a location of the sensor within a patient.

### Example 19

A method of applying suction in a patient, the method comprising: (a) inserting a guidewire into an elastic guidewire channel of a suction instrument to thereby expand the guidewire channel, wherein the guidewire channel defines a lumen that is resiliently expandable such that the lumen expands in response to receiving the guidewire; (b) inserting a cannula of the suction instrument into a patient, wherein the elastic guidewire channel is secured to the cannula; (c) identifying a location of the cannula in the patient based on data from a sensor in the guidewire; (d) positioning a distal end of the cannula at a target site in the patient based on the identified location based on data from the sensor in the guidewire; and (e) applying suction at the target site via the cannula.

### Example 20

The method of Example 19, wherein the act of inserting the cannula into the patient comprises inserting the cannula into a nasal cavity of the patient.

### V. Miscellaneous

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Versions of the devices disclosed herein can be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, versions of the device may be disassembled, and any number of the particular pieces or parts of the device may be selectively replaced or removed in any combination. Upon cleaning and/or replacement of particular parts, versions of the device may be reassembled for subsequent use either at a reconditioning facility, or by a surgical team immediately prior to a surgical procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be processed before surgery. First, a new or used instrument may be obtained and if necessary cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, or high-energy electrons. The radiation may kill bacteria on the instrument and in the container. The sterilized instrument may then be stored in the sterile container. The sealed container may keep the instrument sterile until it is opened in a surgical facility. A device may also be sterilized using any other technique known in the art, including but not limited to beta or gamma radiation, ethylene oxide, or steam.

Having shown and described various versions of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus comprising:
(a) a cannula including:
(i) a proximal end,
(ii) a distal end, and
(iii) a first lumen extending from the proximal end to the distal end;
(b) a suction port that is configured to communicate suction to the distal end of the cannula via the first lumen; and
(c) a guidewire channel including:
(i) an open proximal end,
(ii) an open distal end, and
(iii) a second lumen extending from the open proximal end to the open distal end, wherein the guidewire channel is securely attached to the cannula such that the second lumen is laterally offset from the first lumen, wherein the guidewire channel is configured to slidably receive a guidewire within the second lumen, wherein the guidewire channel is formed of an elastic material such that the guidewire channel is configured to resiliently expand the second lumen outwardly to an expanded state in response to slidably receiving a guidewire therein.

2. The apparatus of claim 1, wherein the guidewire channel is resiliently biased toward an inwardly contracted state.

3. The apparatus of claim 2, wherein the second lumen is configured to have a smaller cross-sectional profile than a guidewire when the guidewire channel is in the inwardly contracted state.

4. The apparatus of claim 1, wherein the guidewire channel is configured to elastically conform to a shape of a guidewire that is slidably received within the second lumen.

5. The apparatus of claim 1, wherein the open distal end of the guidewire channel is positioned adjacent to the distal end of the cannula.

6. The apparatus of claim 1, wherein the cannula further includes a preformed bend formed between the proximal end and the distal end.

7. The apparatus of claim 6, wherein the open proximal end of the guidewire channel is positioned adjacent to the preformed bend, or distal to the preformed bend.

8. The apparatus of claim 6, wherein the guidewire channel further includes a bend formed between the open proximal end and the open distal end, wherein the bend is positioned adjacent to the preformed bend of the cannula.

9. The apparatus of claim 1, wherein the first lumen defines a longitudinal axis, wherein the second lumen is oriented parallel relative to the longitudinal axis.

10. The apparatus of claim 1, wherein the cannula is rigid.

11. The apparatus of claim 1, further comprising a grip secured to the proximal end of the cannula, wherein the grip defines a third lumen providing a path for communication of suction from the suction port to the first lumen.

12. The apparatus of claim 11, wherein the guidewire channel is oriented obliquely relative to the third lumen.

13. The apparatus of claim 1, further comprising a guidewire configured to fit in the second lumen.

14. The apparatus of claim 13, wherein the guidewire includes a navigation sensor configured to communicate with an image guided surgery system.

15. An apparatus comprising:
(a) a cannula including:
(i) a proximal end,
(ii) a distal end, and
(iii) a first lumen extending from the proximal end to the distal end;
(b) a guidewire channel securely attached to the cannula, wherein the guidewire channel defines a second lumen laterally offset from the first lumen, wherein the guidewire channel is configured to resiliently bias the second lumen inwardly to a contracted state; and
(c) a navigation guidewire configured to fit in the second lumen such that the second lumen is operable to expand outwardly to an expanded state, wherein the navigation guidewire includes a sensor configured to generate data based on a location of the sensor within a patient;
wherein the first lumen is configured to provide suction through the distal end while the navigation guidewire is disposed in the second lumen.

16. The apparatus of claim 15, wherein the guidewire channel is formed of an elastic material to provide the resilient bias.

17. The apparatus of claim 15, further comprising an image guided surgery system in communication with the sensor of the guidewire, wherein the image guided surgery system is configured to process the data from the sensor to determine a location of the sensor within a patient.

18. A method of applying suction in a patient, the method comprising:
(a) inserting a guidewire into an elastic guidewire channel of a suction instrument to thereby expand the guidewire channel, wherein the guidewire channel defines a lumen that is resiliently expandable such that the lumen expands in response to receiving the guidewire;
(b) inserting a cannula of the suction instrument into a patient, wherein the elastic guidewire channel is secured to the cannula;
(c) identifying a location of the cannula in the patient based on data from a sensor in the guidewire;
(d) positioning a distal end of the cannula at a target site in the patient based on the identified location based on data from the sensor in the guidewire; and
(e) applying suction at the target site via the cannula.

19. The method of claim 18, wherein the act of inserting the cannula into the patient comprises inserting the cannula into a nasal cavity of the patient.
